# EUROPEAN PATENT SPECIFICATION

(11) **EP 4 454 693 B1**
(45) Date of publication and mention of the grant of the patent: **11.11.2026**
(21) Application number: 21968836.3
(22) Date of filing: 21.12.2021
(51) Int. Cl.: A61M 25/00, A61M 25/06, A61M 25/09

(54) **MEDICAL INSTRUMENT**
MEDIZINISCHES INSTRUMENT
INSTRUMENT MÉDICAL

(43) Date of publication of application: 30.10.2024
(73) Proprietor: ASAHI INTECC CO., LTD., Seto-shi, Aichi, 489-0071 (JP)
(72) Inventor: SHIRAISHI, Ryuya, Seto-shi, Aichi 489-0071 (JP); FUSEYA, Yukihiro, Seto-shi, Aichi 489-0071 (JP); TSUZUKU, Marina, Seto-shi, Aichi 489-0071 (JP); KURITA, Daisuke, Seto-shi, Aichi 489-0071 (JP)
(74) Representative: Winter, Brandl - Partnerschaft mbB
(86) International application number: PCT/JP2021/047209
(87) International publication number: WO 2023/119399

(56) References cited:
- EP-B1- 1 680 172
- WO-A1-2017/110757
- WO-A1-2019/211903
- JP-A- 2004 321 838
- US-A- 5 879 342
- US-A1- 2001 016 728
- US-A1- 2008 004 546
- US-A1- 2021 046 288
- US-A1- 2021 069 480
- US-A1- 2021 069 480

## Description

### TECHNICAL FIELD

The present invention relates to a medical instrument.

### BACKGROUND ART

For example, medical instruments such as dilators and catheters are known as instruments that treat an affected area within a body cavity, or that directly inject drugs into a body cavity (see for example US 2021/0069480 A1).

Such medical instruments are delivered to a treatment site, for example, while being guided by a guide wire that has been inserted into a body cavity in advance. As a result, in order to allow the medical instrument to be smoothly advanced and retracted in the body cavity along the guide wire that has been inserted into the inner cavity, the medical instrument is required to have excellent sliding properties with respect to the guide wire (see US 2021/0046288 A1, US 2008/0004546 A1, US 2001/016728 A and EP 1 680 172 B1, which relate to guide wires).

In order to enhance the sliding properties, for example, proposed is a medical instrument (catheter tube) provided with an inner layer tube made of thermoplastic resin formed inside a braid formed using a metallic element wire, and which is formed having fine irregularities on an inner surface of the inner layer tube to reduce the frictional resistance with a guide wire or the like (for example, see JP 2000 - 225 196 A).

### SUMMARY OF INVENTION

### TECHNICAL PROBLEM

However, although a medical instrument such as that described above has excellent sliding properties with respect to a guide wire, because the entire braid is covered with thermoplastic resin, the flexibility of the medical instrument tends to decrease due to an increase in rigidity. As a result, when the medical instrument is inserted into a curved body cavity, there is a concern that a decrease in the ability to follow the body cavity may occur, thereby inhibiting smooth advancement

The present invention has been made based on the above circumstances, and an object of the present invention is to provide a medical instrument that is capable of achieving enhanced sliding properties with respect to a device, such as a guide wire to be inserted into an inner cavity, while maintaining flexibility.

### SOLUTION TO PROBLEM

This object is solved by the subject matter of the independent claim. Further aspects are disclosed in the dependent claims.

Note that, in the present specification, a "distal end side" refers to a direction along the long axis direction of the coil body, and a direction in which the medical instrument advances deeper into the body cavity (distal direction). Furthermore, a "proximal end side" refers to a direction along the long axis direction of the coil body, and is in the opposite direction to the distal end side (proximal direction). In addition, a "distal end" refers to an end portion of any member or part on the distal end side, and a "proximal end" refers to an end portion of any member or part on the proximal end side. Moreover, a "winding wire" refers to a wire-shaped member that is spirally wound in order to form the coil body. Also, "substantially annular" is a concept that includes both a ring shape and a spiral shape. In addition, "protrusions intermittently disposed" refers to a state in which, in any cross-section including the long axis of the coil body, the protrusions are not continuous over the entire inner cover portion along the long axis direction.

### ADVANTAGEOUS EFFECTS OF INVENTION

The present invention enables a medical instrument to be provided that is capable of achieving enhanced sliding properties with respect to a device, such as a guide wire to be inserted into an inner cavity, while maintaining flexibility.

### BRIEF DESCRIPTION OF DRAWINGS

FIG. 1 is a schematic view showing an entirety of a first reference embodiment.
FIG. 2 is a schematic cross-sectional view showing an enlarged part of FIG. 1.
FIG. 3A is a schematic cross-sectional view showing an example of a method of forming a cover member.
FIG. 3B is a schematic cross-sectional view showing an example of a non-claimed method of forming a cover member.
FIG. 3C is a schematic cross-sectional view showing an example of a non-claimed method of forming a cover member.
FIG. 3D is a schematic cross-sectional view showing an example of a non-claimed method of forming a cover member
FIG. 3E is a schematic cross-sectional view showing an example of a non-claimed method of forming a cover member
FIG. 3F is a schematic cross-sectional view showing an example of a non-claimed method of forming a cover member
FIG. 3G is a schematic cross-sectional view showing an example of a non-claimed method of forming a cover member
FIG. 4 is a schematic cross-sectional view showing a partially enlarged second embodiment.
FIG. 5 is a schematic cross-sectional view showing an enlarged part of a third embodiment partially enlarged.
FIG. 6 is a schematic view showing an entirety of a fourth embodiment.
FIG. 7 is a schematic cross-sectional view showing an enlarged part of FIG. 6.

### DESCRIPTION OF EMBODIMENTS

A medical instrument of the present disclosure includes: a hollow coil body, which is formed by spirally winding a winding wire; and a cover member with which the winding wire of the coil body is covered, wherein the cover member has an outer cover portion that covers an outer peripheral surface of the coil body, and an inner cover portion that covers an inner peripheral surface of the coil body and that is coupled to the outer cover portion through intervals between adjacent turns of the winding wire of the coil body the inner cover portion has a protrusion that protrudes toward a radially inner side of the coil body beyond a common inscribed line of the winding wire in a long axis direction of the coil body, and the protrusion is formed in a substantially annular shape around a long axis of the coil body, and is disposed intermittently along the long axis direction of the coil body.

Hereinafter, first reference embodiment and second to fourth embodiments will be described with reference to the drawings. However, the present disclosure is not limited to the embodiments described in the drawings. Furthermore, the dimensions of the parts shown in the drawings are dimensions shown to facilitate the understanding of the contents of the implementation, and do not necessarily correspond to the actual dimensions. In the cross-sectional views, the outer cover portion and the inner cover portion are shown as a single region for convenience.

### [First Reference Embodiment (not covered by the present claims)]

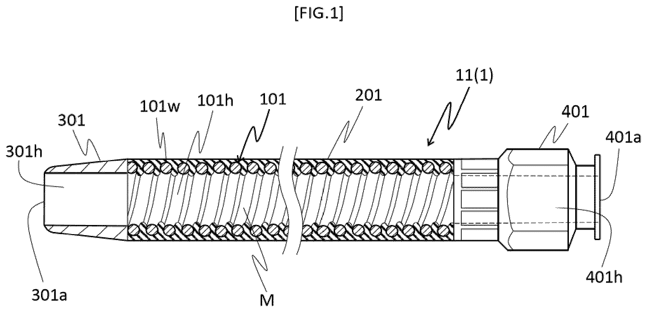

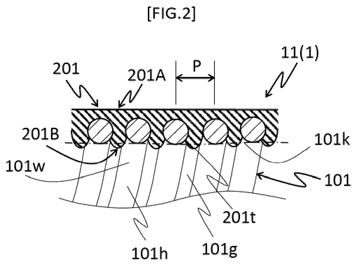

FIGS. 1 and 2 are schematic diagrams showing a first reference embodiment. In the present reference embodiment, a catheter 11 will be described as an example of a medical instrument 1. As shown in FIG. 1, the medical instrument 1 (catheter 11) is schematically configured by a coil body 101, a cover member 201, a distal tip 301, and a proximal portion 401. Note that, in FIG. 1, the coil body 101, the cover member 201, and the distal tip 301 are illustrated as cross-sectional shapes, and the proximal portion 401 is illustrated as a side surface shape.

The coil body 101 is a hollow member, which is formed by spirally winding a winding wire 101w at a pitch P around a long axis of the catheter 11, and has an inner cavity 101h on the inside. Specifically, the coil body 101 may adopt, for example, a single-thread coil body in which a single winding wire is wound in a single thread, a multi-thread coil body in which two or more winding wires are wound in multiple threads, or a combined body formed of a single-thread coil and a multi-thread coil.

### Examples of the winding wire 101w include a solid wire (one single wire) and a twisted wire (a bundled group of wires formed by twisting together two or more single wires in advance).

The material of the winding wire constituting the coil body 101 preferably has, due to being inserted into a body cavity, antithrombogenicity, flexibility and biocompatibility. Examples of the material of the winding wire 101w constituting the coil body 101 include resin materials such as polyamide resins, polyolefin resins, polyester resins, polyurethane resins, silicone resins, and fluororesins; and metallic materials such as stainless steel (such as SUS316) and superelastic alloys (such as nickel-titanium alloy).

The cover member 201 is a member that covers the winding wire 101w of the coil body 101. As shown in FIG. 2, the cover member 201 is configured to include, for example, an outer cover portion 201A and an inner cover portion 201B.

The outer cover portion 201A is a part that covers an outer peripheral surface (the surface on the side facing the outside) of the coil body 101. The outer cover portion 201A may cover only part of the outer peripheral surface of the coil body 101, or may cover the entire outer peripheral surface. The surface (outer peripheral surface) of the outer cover portion 201A may, for example, be formed flat to avoid damaging the tissue of the wall of the body cavity into which the catheter 11 is inserted.

The inner cover portion 201B is a part that covers an inner peripheral surface (surface on the side facing the inner cavity 101h) of the coil body 101, and that is coupled to the outer cover portion 201A through intervals between adjacent turns of the winding wire 101w of the coil body 101. Specifically, for example, the inner cover portion 201B is integrally connected to the outer cover portion 201A through gaps 101g formed between adjacent turns of the winding wire 101w constituting the coil body 101. The gaps 101g may be formed between all adjacent turns of the winding wire 101w, or only formed between some adjacent turns of the winding wire 101w. Furthermore, the inner cover portion 201B and the outer cover portion 201A may be coupled at all of the gaps 101g, or may be coupled at only some of the gaps 101g.

Examples of the material constituting the outer cover portion 201A and the inner cover portion 201B mentioned above include resin materials such as polyamide, polyamide elastomer, polyester, polyurethane, and polytetrafluoroethylene (PTFE).

Note that the material constituting the outer cover portion 201A and the material constituting the inner cover portion 201B may be the same, or different. When the outer cover portion 201A and the inner cover portion 201B are the same material, the outer cover portion 201A and the inner cover portion 201B can be strongly coupled due to improved compatibility. Furthermore, when polytetrafluoroethylene (PTFE) is used for the inner cover portion 201B, the slidability with respect to a guide wire or the like inserted into the inner cavity 101h can be further enhanced.

The surface (inner peripheral surface) of the inner cover portion 201B is, as shown in FIG. 2, formed having protrusions 201t that protrude toward a radially inner side of the coil body 101 beyond a common inscribed line 101k of the winding wire 101w in the long axis direction of the coil body 101. The protrusions 201t are formed in a substantially annular shape around the long axis of the coil body 101, and be disposed intermittently along the long axis direction of the coil body 101.

Here, examples of specific modes of the protrusions 201t being disposed intermittently along the long axis direction include a mode in which two or more protrusions 201t formed in a ring shape around the long axis of the coil body 101 are arranged while being spaced apart in the long axis direction, and a mode in which protrusions 201t formed along the gaps 101g are arranged in a spiral shape while being spaced apart in the long axis direction. When the protrusions 201t are formed in a spiral shape, the protrusions 201t may be configured by a single continuous part along the gaps 101g, or may be configured by two or more independent parts. The pitch in the long axis direction between adjacent protrusions 201t that have been disposed intermittently may be regular (for example, a constant pitch), or irregular.

The protrusions 201t of the present reference embodiment protrude to the inner side of the common inscribed line 101k of the winding wire 101w (lower side of the page), while also being independent (spaced apart) from each other in the long axis direction of the coil body 101, and are formed so as to be continuous in a spiral shape along the gaps 101g of the winding wire 101w in a spiral direction of the coil body 101.

The shape of the surface (inner peripheral surface) of the protrusions 201t is not particularly limited, as long as the effects of the present disclosure are not impaired. In the present reference embodiment, the protrusions 201t are formed so as to be curved in a convex shape toward the radially inner side of the coil body 101. As a result, the contact resistance between a device such as a guide wire that is inserted inside and the inner cover portion 201B can be reduced, and the sliding properties of the inserted device can be further enhanced.

Here, the non-claimed method of forming the cover member 201 will be described. Note that the non-claimed method of forming the cover member 201 is not limited to the following method. Here, a non-claimed method will be described in which the outer cover portion 201A and the inner cover portion 201B are the same material, and the cover member 201 is formed using a heat shrink tube.

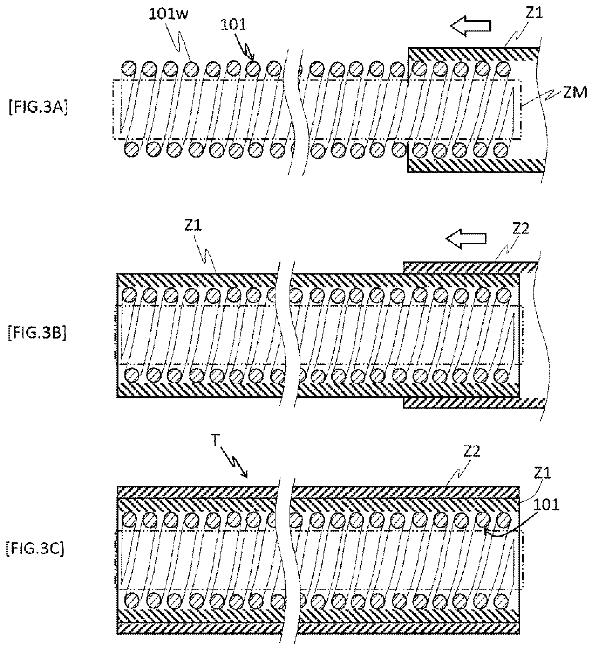

First, using the coil body 101 formed by the winding wire 101w, a resin tube Z1 for forming the cover member is placed so as to cover the entire outer peripheral surface of the coil body 101 (see FIG. 3A). Then, a heat shrink tube Z2 is placed so as to cover the entire outer peripheral surface of the resin tube Z1 (see FIG. 3B). As a result, a three-layer structure T consisting of, in order from the inner side, the coil body 101, the resin tube Z1, and the heat shrink tube Z2 is formed as shown in FIG. 3C. Note that, in order to ensure that the coil body 101 after forming the cover member 201 has the inner cavity 101h with a predetermined inner diameter, a mandrel ZM with a predetermined outer diameter may be inserted in advance into the inner cavity of the coil body 101 constituting the three-layer structure T (see the mandrel ZM illustrated as a virtual line in FIGS. 3A to 3F).

As the heat shrink tube Z2, it is possible to select a tube that shrinks with a predetermined pressure at a temperature in which the material of the resin tube Z1, that is, the material constituting the cover member 201, softens or melts (a temperature greater than or equal to the glass transition point). The material constituting the heat shrink tube Z2 depends on the material of the cover member 201, but examples include synthetic resins such as polyolefin resins, polyester resins, nylon resins, silicone resins, and fluorine resins. The material of the heat shrink tube Z2 may be used alone or in combination of two or more types.

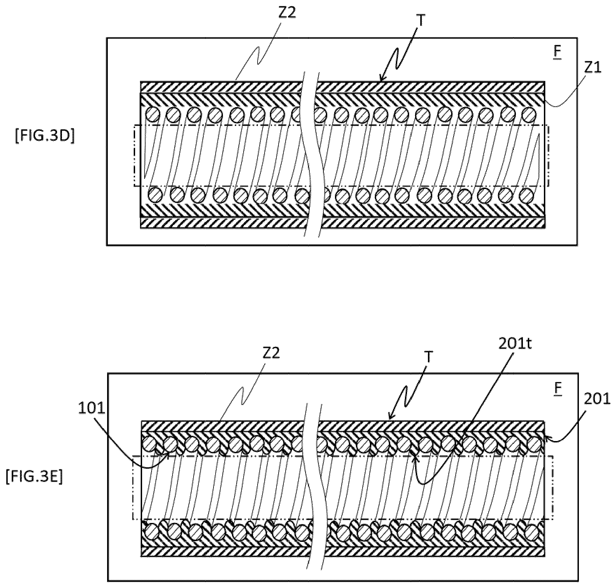

Next, the three-layer structure T is placed in a heating furnace F (see FIG. 3D), and heated at a predetermined temperature for a predetermined time. Here, the predetermined temperature and the predetermined time refer to a temperature and time that allow the material constituting the resin tube Z1 to soften or melt, and for a portion of the softened or melted resin tube Z1 to be pushed out through the gaps 101g into the inner cavity of the coil body 101 due to the shrinkage of the heat shrink tube Z2 to form the protrusions 201t.

The heated three-layer structure T, as shown in FIG. 3E, gradually shrinks in diameter due to a contraction force of the heat shrink tube Z2 that is positioned at the outermost periphery, and the protrusions 201t are formed as a result of a portion of the softened or melted resin tube Z1 being pushed out through the gaps 101g into the inner cavity of the coil body 101. At this time, the surface (inner peripheral surface) of the protrusions 201t has a convex curved shape toward the radially inner side of the coil body 101 due to the surface tension of the softened or melted resin

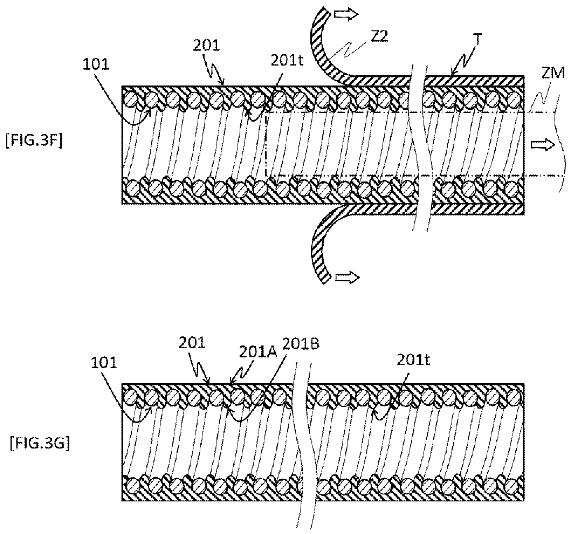

Next, after cooling the three-layer structure T, whose heating has been completed, to room temperature, by removing the heat shrink tube Z2 (and the mandrel ZM) as shown in FIG. 3F, the cover member 201 that covers the coil body 101 as shown in FIG. 3G is formed. Note that, of the cover member 201 that has been formed, the part covering the outer peripheral surface of the coil body 101 forms the outer cover portion 201A, and the part covering the inner peripheral surface of the coil body 101 forms the inner cover portion 201B.

The distal tip 301 is a member provided on a distal end portion of the catheter 11. Specifically, the distal tip 301 may be formed, for example, having a shape in which the distal end portion is rounded toward the distal end side such that the catheter 11 can easily move through the body cavity. The distal tip 301 has an opening 301a on a distal end, and has an inner cavity 301h that communicates with the inner cavity 101h of the coil body 101.

The material constituting the distal tip 301 preferably has antithrombotic properties, biocompatibility, as well as flexibility so as to reduce impact toward the body cavity and the like. Examples of such materials include resin materials such as polyurethane and polyurethane elastomer.

Examples of the non-claimed method of coupling the distal tip 301 and the coil body 101 include a non-claimed method of bonding a proximal end portion of the distal tip 301 to a distal end portion of the winding wire 101w constituting the coil body 101 by welding, or by using an adhesive.

The proximal portion 401 is a member that is used by an operator to operate the catheter 11. The proximal portion 401 is connected to a proximal end portion of the coil body 101. The proximal portion 401 includes, for example, an opening 401a on a proximal end, and includes an inner cavity 401h that communicates with the inner cavity 101h of the coil body 101. The shape of the proximal portion 401 is not particularly limited as long as the effects of the present invention are not impaired, and may be formed, for example, in a shape that is easily operated by the operator.

The inner cavity 301h of the distal tip 301, the inner cavity 101h of the coil body 101, and the inner cavity 401h of the proximal portion 401 form a lumen M. For example, a device such as a guide wire is inserted into the lumen M.

Next, a usage mode of the medical instrument 1 will be described. Here, the medical instrument 1 (catheter 11) is a guiding catheter (hereinafter, also referred to as "guiding catheter 11"), and a procedure will be illustrated in which a balloon catheter is inserted into the lumen M of the guiding catheter while dilating a constricted part that has formed in coronary artery of the heart.

Prior to using the guiding catheter 11, a guide wire A is firstly inserted into a blood vessel, and the distal end is delivered to a position near the entrance of a coronary artery of the heart. Then, the proximal end of the guide wire A is inserted into the opening 301a of the guiding catheter 11, and the guiding catheter 11 is advanced along the guide wire A while being pushed into the blood vessel such that the distal end reaches the entrance of the coronary artery of the heart. At this time, the guiding catheter 11 is fed while following the curvature of the blood vessel

Next, after removing the guide wire A to the outside of the body, a thin guide wire B for a balloon catheter is inserted into the guiding catheter 11 through the opening 401a, and a distal end of the guide wire B is made to reach a position beyond the constricted part through the guiding catheter 11. Then, a proximal end of the guide wire B is inserted into a distal end opening of the balloon catheter, and the balloon catheter is pushed forward along the guide wire B up to the inner side of the constricted part. Next, the constricted part is dilated by inflating the balloon of the balloon catheter. After dilating the constricted part, the balloon catheter, the guide wire B, and the guiding catheter 11 are removed in this order to the outside of the body, which enables the procedure using the guiding catheter 11 to be completed.

As described above, because the medical instrument 1 (catheter 11) has the above configuration, it is possible to reduce the contact resistance between a device such as a guide wire that is inserted inside and the inner cover portion 201B, and it is possible to enhance the sliding properties with respect to a device inserted into the inner cavity 101h, while maintaining the flexibility of the coil body 101. Note that, it is thought that the coil body 101 is capable of maintaining flexibility because, when an external force in a direction orthogonal to the long axis direction is applied to the catheter 11, the coil body becomes more easily bent in the orthogonal direction from the parts between adjacent protrusions, which have a substantially annular shape and are intermittently formed spaced apart from each other (the protrusions formed in a bellows shape suppress an increase in the rigidity of the catheter).

### [Second Embodiment]

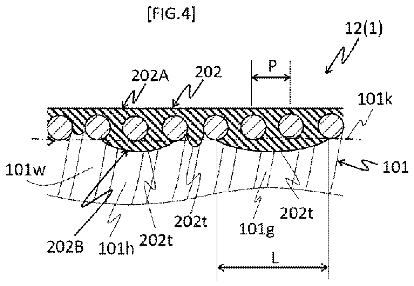

FIG. 4 is a schematic cross-sectional view showing a second embodiment partially enlarged. The medical instrument 1 (catheter 12) is schematically configured by a coil body 101, a cover member 202, a distal tip 301 (not illustrated), and a proximal portion 401 (not illustrated). The catheter 12 differs from the first reference embodiment in that the cover member 202 is provided. Note that, because the coil body 101, the distal tip 301, and the proximal portion 401 are the same as those of the first reference embodiment, the same parts are designated by the same reference numerals, and the detailed description thereof will be omitted. Furthermore, except for the configuration of the cover member 202 that is described below, the configuration of the cover member is the same as in the first reference embodiment.

The cover member 202 is a member that covers the winding wire 101w of the coil body 101. The cover member 202 is configured to include, for example, an outer cover portion 202A and an inner cover portion 202B. The outer cover portion 202A is a part that covers the outer peripheral surface of the coil body 101. The inner cover portion 202B is a part that covers the inner peripheral surface of the coil body 101, and that is coupled to the outer cover portion 202A through intervals between adjacent turns of the winding wire 101w of the coil body 101. The inner cover portion 202B has protrusions 202t that protrude toward the radially inner side of the coil body 101 beyond the common inscribed line 101k of the winding wire 101w in the long axis direction of the coil body 101, and the protrusions 202t are formed in a substantially annular shape around the long axis of the coil body 101, and are disposed intermittently along the long axis direction of the coil body 101.

The length of the protrusions 202t in the long axis direction is formed such that protrusions are included that are 2 times or more the pitch of adjacent turns of the winding wire 101w. In the present embodiment, as shown in FIG. 4, the inner cover portion 202B is illustrated that includes protrusions 202t whose length L in the long axis direction is a length that is approximately 3 times the pitch P of the winding wire 101w. Furthermore, in the catheter 12, the protrusions 202t are each formed so as to be curved in a convex shape toward the radially inner side of the coil body 101. In addition, the protrusions 202t are disposed intermittently along the long axis direction of the coil body 101, that is, the protrusion 202t and the adjacent protrusions 202t are spaced apart in the long axis direction of the coil body 101

As the non-claimed method of forming the inner cover portion 202B, for example, in the non-claimed method of forming the cover member 202 described in the first reference embodiment, it is possible to employ a method in which the three-layer structure T is heated at a predetermined temperature for a predetermined time while adjusting the pressure at the time of heat shrinkage by appropriately selecting the type of heat shrink tube Z2 used for the three-layer structure T. Moreover, the material constituting the cover member 202 may be appropriately selected to adjust the fluidity during heating. Consequently, it is possible to control the amount of the softened or melted resin tube Z1 that is pushed out through the gaps 101g into the inner cavity, and as a result, it is possible for the protrusions 202t to be formed having a desired length. Note that the protrusions 202t having a convex curved shape toward the radially inner side of the coil body 101 are formed due to the surface tension of the softened or melted resin.

As described above, in the catheter 12, because the length L of the protrusions 202t in the long axis direction is formed to include protrusions that are 2 or more times the pitch P of adjacent turns of the winding wire 101w, as a result of the inner cover portion 202B covering the entire winding wire 101w that is adjacent to the gaps 101g, it is possible to reduce the detachment of the protrusions 202t from the winding wire 101w, even if the inner cover portion 202B and the outer cover portion 202A become separated for some reason.

### [Third Embodiment]

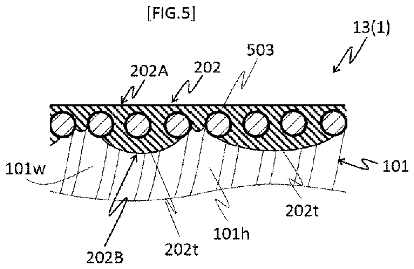

FIG. 5 is a schematic cross-sectional view showing a third embodiment partially enlarged. As shown in FIG. 5, the medical instrument 1 (catheter 13) is schematically configured by a coil body 101, a base layer 503, a cover member 202, a distal tip 301 (not illustrated), and a proximal portion 401 (not illustrated). The catheter 13 differs from the second embodiment in that the base layer 503 is provided. Note that, because the coil body 101, the cover member 202, distal tip 301, and the proximal portion 401 are the same as those of the second embodiment, the same parts are designated by the same reference numerals, and the detailed description thereof will be omitted.

The base layer 503 is a layered part that is disposed so as to be in contact with the winding wire 101w and the cover member 202, and is provided to enhance the adhesion between the winding wire 101w and the cover member 202.

Examples of the material constituting the base layer 503 include acrylic resins, but in addition to this, urethane resins and epoxy resins can also be selected.

The thickness of the base layer 503 is preferably about 1 µm, for example, but the film thickness can be changed as appropriate depending on the specifications.

As described above, because the catheter 13 is provided with the base layer 503 so as to make contact with winding wire 101w and the cover member 202, it is possible to enhance the adhesion between the winding wire 101w and the cover member 202, and it is possible to reduce the detachment of the protrusions 202t from the winding wire with more certainty, even if the inner cover portion 202B and the outer cover portion 202A become separated for some reason.

### [Fourth Embodiment]

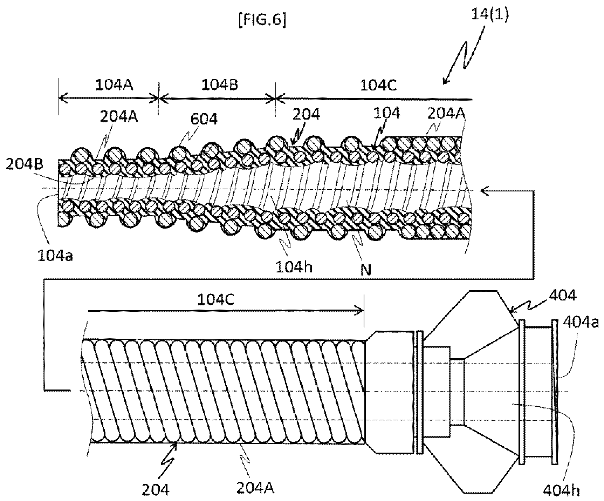

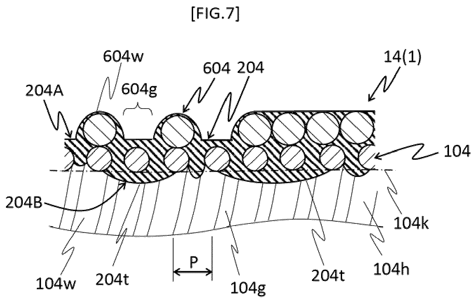

FIGS. 6 and 7 are schematic diagrams showing a fourth embodiment. In the present embodiment, a dilator 14 will be described as an example of the medical instrument 1. As shown in FIG. 6, the medical instrument 1 (dilator 14) is schematically configured by a coil body 104, a spirally-arranged protruding portion 604, a cover member 204, and a proximal portion 404. The dilator 14 differs from the first reference embodiment in that it includes the coil body 104, the spirally-arranged protruding portion 604, the cover member 204, and the proximal portion 404. Note that, except for the configurations of the coil body 104, the cover member 204, and the proximal portion 404 that are described below, the configurations of each of the coil body, the cover member, and the proximal portion are the same as in the first reference embodiment. Furthermore, in FIG. 6, the distal end portion of the dilator 14 is illustrated as a cross-sectional shape, and the proximal end portion of the dilator 14 is illustrated as a side surface shape.

The coil body 104 is a hollow member, which is formed by winding a winding wire 104w in a spiral shape. The coil body 104 can be formed, for example, by winding the winding wire 104w in a spiral shape at a pitch P around a long axis of the dilator 14. The coil body 104 includes an inner cavity 104h having an opening 104a at a distal end.

The coil body 104 of the present embodiment includes a tapered portion 104B, a distal end portion 104A, and a main body portion 104C. The tapered portion 104B is a part whose outer diameter at a distal end is smaller than the outer diameter at a proximal end. The distal end portion 104A has a proximal end positioned at the distal end of the tapered portion 104B, and is a part having a constant outer diameter that is provided extending toward the distal end side in an axial direction of the tapered portion 104B. The main body portion 104C has a distal end positioned at the proximal end of the tapered portion 104B, and is a part having a constant outer diameter that is provided extending toward the proximal end side in the axial direction of the tapered portion 104B.

The spirally-arranged protruding portion 604 is provided on an outer peripheral surface of the coil body 104, and is a member that protrudes toward a radially outer side of the coil body 104. The spirally-arranged protruding portion 604 has gaps 604g in adjacent portions along a long axis direction of the coil body 104. The spirally-arranged protruding portion 604 may be continuously formed along the long axis direction, or may be intermittently formed. Furthermore, the spirally-arranged protruding portion 604 may be a single-thread protruding portion, or may be a multi-thread protruding portion.

The spirally-arranged protruding portion 604 is provided on at least an outer periphery of the tapered portion. In the present embodiment, the spirally-arranged protruding portion 604 is formed on the outer periphery of the distal end portion 104A, the tapered portion 104B, and the main body portion 104C.

The spirally-arranged protruding portion 604 can be formed, for example, by winding a wire 604w (single wire) around the outer periphery of the coil body 104.

Because the spirally-arranged protruding portion 604 is inserted into a body cavity, the material constituting the spirally-arranged protruding portion 604 has antithrombogenicity, flexibility and biocompatibility. Examples of the material constituting the spirally-arranged protruding portion 604 include resin materials such as polyamide resins, polyolefin resins, polyester resins, polyurethane resins, silicone resins, and fluororesins; and metallic materials such as stainless steel (such as SUS316) and superelastic alloys (such as nickel-titanium alloy).

Examples of the non-claimed method of coupling the coil body 104 and the spirally-arranged protruding portion 604 include a non-claimed method of brazing the two parts at appropriate locations (such as a contact part or an end portions) using a brazing material, welding methods, and bonding methods using an adhesive.

The cover member 204 is a member that covers the winding wire 104w of the coil body 104. As shown in FIG. 7, the cover member 204 is configured to include, for example, an outer cover portion 204A and an inner cover portion 204B. The outer cover portion 204A is a part that covers the outer peripheral surface of the coil body 104. The inner cover portion 204B is a part that covers the inner peripheral surface of the coil body 104, and that is coupled to the outer cover portion 204A through intervals between adjacent turns of the winding wire 104w of the coil body 104. The inner cover portion 204B has protrusions 204t that protrude toward the radially inner side of the coil body 104 beyond a common inscribed line 104k of the winding wire 104w in the long axis direction of the coil body 104, and the protrusions 204t are formed in a substantially annular shape around the long axis of the coil body 104, and are disposed intermittently along the long axis direction of the coil body 104.

The outer cover portion 204A may be provided so as to cover the spirally-arranged protruding portion 604, or may be provided so as to not cover the spirally-arranged protruding portion 604. When the outer cover portion 204A covers the spirally-arranged protruding portion 604, the part of the spirally-arranged protruding portion 604 that is covered by the outer cover portion 204A may be part or all of the outer peripheral surface of the spirally-arranged protruding portion 604.

The outer cover portion 204A of the present embodiment is provided so as to cover at least part of the outer peripheral surface of the spirally-arranged protruding portion 604 while spanning the coil body 104 and the spirally-arranged protruding portion 604. As a result, the coupling strength between the coil body 104 and the spirally-arranged protruding portion 604 can be further enhanced.

The proximal portion 404 is a member that is used by an operator to operate the dilator 14. The proximal portion 404 is connected to a proximal end portion of the coil body 104 and a proximal end portion of the spirally-arranged protruding portion 604. The proximal portion 404, for example, includes an opening 404a on a proximal end, and includes an inner cavity 404h that communicates with the inner cavity 104h of the coil body 104. The shape of the proximal portion 404 is not particularly limited as long as the effects of the present invention are not impaired, and may be formed, for example, in a shape that is easily operated by the operator.

The inner cavity 104h of the coil body 104 and the inner cavity 404h of the proximal portion 404 form a lumen N. For example, a device such as a guide wire is inserted into the lumen N.

Next, a usage mode of the medical instrument 1 (dilator 14) will be described. Here, a procedure will be illustrated in which a hole (a portion to be expanded) is pierced through a wall of an organ such as a stomach wall, and the dilator 14 is used to expand the pierced hole.

First, a hole (a portion to be expanded) is formed by piercing the wall of the organ using an introduction needle (not illustrated). Then, after inserting a guide wire (not illustrated) into an inner cavity of the introduction needle, the introduction needle is removed to the outside of the body along the guide wire.

Next, a proximal end of the guide wire is inserted into the inner cavity 104h through the opening 104a of the dilator 14, and the distal end of the dilator 14 is pushed forward to a position immediately before the pierced hole. Then, the distal end portion 104A of the coil body 104 is inserted into the hole, and the dilator 14 is moved forward such that the tapered portion 104B comes into contact with the inner wall of the hole. Next, while rotating the proximal portion 404, the spirally-arranged protruding portion 604 provided on the outer peripheral surface of the tapered portion 104B is engaged with the inner wall of the hole. At this time, the coil body 104 moves forward as a result of a screw action of the spirally-arranged protruding portion 604, and the hole is gradually expanded by the outer peripheral surface of the tapered portion 104B. When the proximal end of the tapered portion 104B passes through the hole, the hole is expanded to a size corresponding to the outer diameter of the proximal end of the tapered portion 104B (which is equivalent to the outer diameter of the main body portion 104C).

Note that, when the hole is expanded due to the operation of the proximal portion 404, the rotation of the coil body 104 and the linear pressing in the long axis direction may be used together.

Then, after expansion of the hole has been completed, by removing the dilator 14 and the guide wire to the outside of the body in this order, the procedure using the dilator 14 is completed.

As described above, in the dilator 14, the spirally-arranged protruding portion 604 that protrudes toward the radially outer side of the coil body 104 is provided on the outer peripheral surface of the coil body 104, and the spirally-arranged protruding portion 604 has the gaps 604g along the long axis direction of the coil body 104. As a result, because of the screw action of the spirally-arranged protruding portion 604 that occurs with the rotation of the coil body 104, the dilator 14 can be easily advanced and retracted in the body cavity with certainty.

Furthermore, in the dilator 14, the coil body 104 has the tapered portion 104B, whose outer diameter at the distal end is smaller than the outer diameter at the proximal end, and the spirally-arranged protruding portion 604 is provided on the outer periphery of the tapered portion 104B. Therefore, it is possible to easily expand a portion to be expanded (such as a pierced hole or a constricted part) in conjunction with the screw action of the spirally-arranged protruding portion 604.

Note that the present invention is not limited to the configurations of the embodiments described above, but is stipulated by the claims, and the present invention is intended to include all modifications within the meaning and scope of the claims.

For example, in the first reference embodiment and second to third embodiments described above, the catheters 11, 12 and 13 provided with the distal tip 301 and the proximal portion 401 have been described. However, a catheter that is not provided with the distal tip and/or proximal portion is also possible.

In addition, in the fourth embodiment described above, the dilator 14 has been described, in which the coil body 104 has the tapered portion 104B, and the spirally-arranged protruding portion 604 is provided on the outer periphery of the tapered portion 104B. However, a medical instrument is also possible in which the spirally-arranged protruding portion is formed on a coil body that does not have a tapered portion (such as a step-shaped coil body, or a coil body in which the outer diameter is constant from the distal end to the proximal end). Furthermore, a dilator is also possible in which the coil body has the tapered portion, and the spirally-arranged protruding portion is formed only on a part other than the tapered portion (such as the distal end portion and/or main body portion).

Moreover, in the fourth embodiment described above, the dilator 14 has been described in which the spirally-arranged protruding portion 604 is provided only on a distal portion of the outer peripheral surface of the coil body in the long axis direction. However, the spirally-arranged protruding portion may be formed across the entire outer peripheral surface of the coil body in the long axis direction.

Furthermore, in the fourth embodiment described above, the dilator 14 provided with the proximal portion 404 has been described. However, a dilator that is not provided with a proximal portion, or a dilator that is provided with a distal tip such as the distal tip 301 is also possible.

### DESCRIPTION OF REFERENCE NUMERALS

1 Medical instrument
11, 12, 13 Catheter
14 Dilator
101, 104 Coil body
101k, 104k Common inscribed line
101w, 104w Winding wire
104B Tapered portion
201, 202, 204 Cover member
201A, 202A, 204A Outer cover portion
201B, 202B, 204B Inner cover portion
201t, 202t, 204t Protrusion
503 Base layer
604 Spirally-arranged protruding portion
604g Gap
L Length of protrusion
P Pitch of winding wire

## Claims

1. A medical instrument (1) into which a device is insertable, the medical instrument (1) comprising:
a hollow coil body (101, 104) formed by spirally winding a winding wire (101w, 104w) and having an inner cavity (101h); and
a cover member (202, 204) with which the winding wire (101w, 104w) of the coil body (101, 104) is covered, wherein
the cover member (202, 204) has an outer cover portion (202A, 204A) that covers an outer peripheral surface of the coil body (101, 104), and an inner cover portion (202B, 204B) that covers an inner peripheral surface of the coil body (101, 104) and that is coupled to the outer cover portion (202A, 204A) through intervals between adjacent turns of the winding wire (101w, 104w) of the coil body (101, 104),
the inner cover portion (202B, 204B) has protrusions (202t, 204t) that protrude toward a radially inner side of the coil body (101, 104) beyond a common inscribed line (101k, 104k) of the winding wire (101w, 104w) in a long axis direction of the coil body (101, 104), and
the protrusions (202t, 204t) are formed in a substantially annular shape around a long axis of the coil body (101, 104), and are disposed intermittently along the long axis direction of the coil body (101, 104), **characterized in that**
the protrusions (202t, 204t) include a protrusion having a length in the long axis direction which is 2 times or more a pitch (P) of adjacent turns of the winding wire (101w, 104w).

2. The medical instrument (1) according to claim 1, wherein
at least one of the protrusions (202t, 204t) is curved in a convex shape toward the radially inner side of the coil body (101, 104).

3. The medical instrument (1) according to claim 1 or 2, comprising
a spirally-arranged protruding portion (604) formed on the outer peripheral surface of the coil body (104) and that protrudes toward a radially outer side of the coil body (104), wherein
the spirally-arranged protruding portion (604) has gaps (604g) along the long axis direction of the coil body (104).

4. The medical instrument (1) according to claim 3, wherein
the spirally-arranged protruding portion (604) is formed by winding a wire (604w) around the outer periphery of the coil body (104).

## Patentansprüche

1. Medizinisches Instrument (1), in das eine Vorrichtung einführbar ist, mit
einem hohlen Spulenkörper (101, 104), der durch spiralförmiges Wickeln eines Wickeldrahts (101w, 104w) gebildet ist und einen inneren Hohlraum (101h) aufweist; und
einem Abdeckelement (202, 204), mit dem der Wickeldraht (101w, 104w) des Spulenkörpers (101, 104) bedeckt ist, wobei
das Abdeckelement (202, 204) einen äußeren Abdeckabschnitt (202A, 204A) aufweist, der eine Außenumfangsfläche des Spulenkörpers (101, 104) bedeckt, sowie einen inneren Abdeckabschnitt (202B, 204B) aufweist, der eine innere Umfangsfläche des Spulenkörpers (101, 104) bedeckt und der mit dem äußeren Abdeckabschnitt (202A, 204A) über Zwischenräume zwischen benachbarten Windungen des Wickeldrahts (101w, 104w) des Spulenkörpers (101, 104) verbunden ist,
der innere Abdeckabschnitt (202B, 204B) Vorsprünge (202t, 204t) aufweist, die in Richtung einer radial inneren Seite des Spulenkörpers (101, 104) über eine gemeinsame Einschreibungslinie (101k, 104k) des Wickeldrahts (101w, 104w) in Längsachsenrichtung des Spulenkörpers (101, 104) ragen, und
die Vorsprünge (202t, 204t) im Wesentlichen ringförmig um eine Längsachse des Spulenkörpers (101, 104) ausgebildet sind und in Abständen entlang der Längsachse des Spulenkörpers (101, 104) angeordnet sind, **dadurch gekennzeichnet, dass**
die Vorsprünge (202t, 204t) einen Vorsprung umfassen, dessen Länge in Längsachsenrichtung das Zweifache oder mehr eines Teilungsabstands (P) benachbarter Windungen des Wickeldrahts (101w, 104w) beträgt.

2. Medizinische Instrument (1) nach Anspruch 1, wobei
mindestens einer der Vorsprünge (202t, 204t) in Richtung der radial inneren Seite des Spulenkörpers (101, 104) konvex gekrümmt ist.

3. Medizinische Instrument (1) nach Anspruch 1 oder 2, umfassend
einen spiralförmig angeordneten Vorsprungs-Abschnitt (604), der an der Außenumfangsfläche des Spulenkörpers (104) ausgebildet ist und zu einer radial äußeren Seite des Spulenkörpers (104) hin vorsteht, wobei
der spiralförmig angeordnete Vorsprungs-Abschnitt (604) entlang der Längsachse des Spulenkörpers (104) Lücken (604g) aufweist.

4. Medizinische Instrument (1) nach Anspruch 3, wobei
der spiralförmig angeordnete Vorsprungs-Abschnitt (604) durch Wickeln eines Drahtes (604w) um den Außenumfang des Spulenkörpers (104) gebildet ist.

## Revendications

1. Instrument médical (1) dans lequel un dispositif peut être inséré, l'instrument médical (1) comprenant :
un corps de bobine creux (101, 104) formé par enroulement en spirale d'un fil d'enroulement (101w, 104w) et présentant une cavité intérieure (101h) ; et
un élément de couverture (202, 204) qui recouvre le fil d'enroulement (101w, 104w) du corps de la bobine (101, 104), dans lequel
l'élément de couverture (202, 204) comporte une partie de couverture extérieure (202A, 204A) qui recouvre une surface périphérique extérieure du corps de la bobine (101, 104), et une partie de couverture intérieure (202B, 204B) qui recouvre une surface périphérique intérieure du corps de la bobine (101, 104) et qui est couplée à la partie de couverture extérieure (202A, 204A) par des intervalles entre des spires adjacentes du fil d'enroulement (101w, 104w) du corps de la bobine (101, 104),
la partie de couverture intérieure (202B, 204B) présente des protubérances (202t, 204t) qui font saillie vers un côté radial intérieur du corps de la bobine (101, 104) au-delà d'une ligne inscrite commune (101k, 104k) du fil d'enroulement (101w, 104w) dans la direction de l'axe longitudinal du corps de la bobine (101, 104), et
les protubérances (202t, 204t) sont formées de manière sensiblement annulaire autour d'un axe longitudinal du corps de la bobine (101, 104) et sont disposées de manière intermittente le long de l'axe long du corps de la bobine (101, 104), **caractérisé en ce que**
les protubérances (202t, 204t) incluent une protubérance présentant une longueur dans la direction de l'axe long qui est 2 fois ou plus un pas (P) de spires adjacentes du fil d'enroulement (101w, 104w).

2. Instrument médical (1) selon la revendication 1, dans lequel
au moins une des protubérances (202t, 204t) est courbée en forme convexe vers le côté radialement intérieur du corps de la bobine (101, 104).

3. Instrument médical (1) selon la revendication 1 ou 2, comprenant
une partie saillante disposée en spirale (604) formée sur la surface périphérique extérieure du corps de la bobine (104) et qui fait saillie vers un côté radial extérieur du corps de la bobine (104), dans lequel
la partie saillante disposée en spirale (604) présente des espaces (604g) le long de l'axe longitudinal du corps de la bobine (104).

4. Instrument médical (1) selon la revendication 3, dans lequel
la partie saillante disposée en spirale (604) est formée en enroulant un fil (604w) autour de la périphérie extérieure du corps de la bobine (104).
